(19) Europäisches Patentamt
European Patent Office
Office européen des brevets

(11) **EP 0 843 821 B1**

(12) **EUROPÄISCHE PATENTSCHRIFT**

(45) Veröffentlichungstag und Bekanntmachung des Hinweises auf die Patenterteilung:
**04.10.2001 Patentblatt 2001/40**

(21) Anmeldenummer: **96917465.5**

(22) Anmeldetag: **30.05.1996**

(51) Int Cl.[7]: **G01N 33/68**

(86) Internationale Anmeldenummer:
**PCT/EP96/02319**

(87) Internationale Veröffentlichungsnummer:
**WO 97/05490 (13.02.1997 Gazette 1997/08)**

(54) **VERFAHREN ZUR CHARAKTERISIERUNG DER GLYCOSYLIERUNG VON GLYCOPROTEINEN SOWIE ZUR IN-VITRO BESTIMMUNG DER BIOVERFÜGBARKEIT VON GLYCOPROTEINEN**

PROCESS FOR CHARACTERISING THE GLYCOSYLATION OF GLYCO-PROTEINS AND FOR THE IN VITRO DETERMINATION OF THE BIO-AVAILABILITY OF GLYCO-PROTEINS

PROCEDE POUR LA CARACTERISATION DE LA GLYCOSYLATION DES GLYCOPROTEINES ET POUR LA DETERMINATION IN VITRO DE LA BIODISPONIBILITE DES GLYCOPROTEINES

(84) Benannte Vertragsstaaten:
**AT BE CH DE DK ES FI FR GB IT LI LU NL PT SE**

(30) Priorität: **26.07.1995 DE 19527054**

(43) Veröffentlichungstag der Anmeldung:
**27.05.1998 Patentblatt 1998/22**

(73) Patentinhaber: **Aventis Pharma Deutschland GmbH**
**65929 Frankfurt am Main (DE)**

(72) Erfinder:
• **HERMENTIN, Peter**
**D-35041 Marburg (DE)**
• **WITZEL, Reinhild**
**D-35041 Marburg (DE)**

(56) Entgegenhaltungen:
• **ANALYTICAL BIOCHEMISTRY, Bd. 203, - 1992 NEW YORK NY USA, Seiten 281-289, XP000576109 P. HERMENTIN ET AL.: "A strategy for the mapping of N-glycans by high-pH anion-exchange chromatography with pulsed amperometric detection" in der Anmeldung erwähnt**
• **ANALYTICAL BIOCHEMISTRY, Bd. 206, 1992, NEW YORK NY USA, Seiten 419-429, XP000576110 P. HERMENTIN ET AL.: "The mapping by high-pH anion-exchange chromatography with pulsed amperometric detection and capillary electrophoresis of the carbohydrate moieties of human plasma alpha-1-acid glycoprotein." in der Anmeldung erwähnt**
• **GLYCOBIOLOGY, Bd. 6, Nr. 2, 1996, OXFORD UK, Seiten 217-230, XP000610494 P. HERMENTIEN ET AL.: "The hypothetical N-glycan charge: a number that characterizes protein glycosylation."**

EP 0 843 821 B1

**Beschreibung**

Gegenstand der vorliegenden Erfindung ist ein Verfahren zur Charakterisierung der Glycosylierung von Glycoproteinen sowie ein in-vitro Verfahren zur Bestimmung der Bioverfügbarkeit von Glycoproteinen, das auf der "hypothetischen Ladungszahl" (im folgenden "Z" genannt) basiert und sowohl für endogene Glycoproteine als auch exogene Glycoproteine eingesetzt werden kann.

Exogene Glycoproteine sind in diesem Zusammenhang z.B. rekombinante, aus Säugerzellen gewonnene therapeutische Glycoproteine (wie z. B. Interleukin 2, Erythropoietin, Gewebsplasminogen-Aktivator oder Antithrombin III). Diese Substanzen haben in den letzten Jahren ein erhebliches Interesse bei wissenschaftlichen, pharmazeutischen und behördlichen Institutionen geweckt.

Endogene Glycoproteine sind in diesem Zusammenhang humane oder nicht humane (z. B. bovine) Serumglycoproteine (wie z. B. humanes $\alpha_1$-saures Glycoprotein, humanes Transferrrin oder bovines Fetuin, aber auch Glycoproteine anderer Spezies, wie z. B. Hühner-Ovomucoid oder Schweine-Thyroglobulin.

Die Erforschung, Entwicklung und Produktion therapeutischer Glycoproteine sowie ihre behördliche bzw. klinische Zulassung erfordert eine aufwendige Analytik im Hinblick auf die in vivo-Halbwertszeit, biologische Sicherheit, Produkt-Definition und Chargenkonsistenz.

In dieser Hinsicht spielt bislang vor allem der Anteil an Sialinsäure- (N-Acetylneuraminsäure, Neu5Ac) als Parameter eine wichtige Rolle, da man davon ausgeht, daß das Vorhandensein bzw. Fehlen von Neu5Ac die Zirkulations-Halbwertszeit eines Glycoproteins im Blut bzw. seine Clearance entscheidend mitbestimmt. Die exakte Untersuchung der Kohlenhydrat-Seitenketten eines Glycoproteins erfordert allerdings eine sehr aufwendige und komplexe Analyse, die ein hohes Maß an Expertise und instrumenteller Infrastruktur verlangt (wie z. B. GC-MS, FAB-MS und hochauflösende 1H-NMR-Spektroskopie).

Vor allem die für die Zulassung therapeutischer Glycoproteine (z. B. EPO) verantwortlichen Behörden verlangen aus Sicherheitsgründen immer noch die sehr aufwendige, zeitraubende, teure aber recht ungenaue Ermittlung der therapeutischen Wirksamkeit des Glycoproteins in Tierexperimenten (in-vivo Assay). Vorteilhafterweise sollte jedoch ein in-vitro Assay zur Verfügung stehen, der zum einen einfach und zuverlässig durchzuführen ist und zum anderen den berechtigterweise hohen Ansprüchen der Zulassungsbehörden genügt.

In Bezug auf die Durchführung von Zusatzbestimmungen ist es bereits in gewissem Maße gelungen, die langwierigen und teuren Methoden der Glyco-Analytik durch standardisierbare chromatographische Verfahren zu ersetzen (Hermentin et al. (1992) *Anal. Biochem.* **203,** 281-289; idem., ibid., (1992) **206,** 419-429). Bislang gab es jedoch keinen Parameter, der den Anforderungen der Zulassungsbehörden als Ersatz für einen in-vivo Assay für die Bioverfügbarkeit eines Glycoproteins genügt hätte.

Der vorliegenden Erfindung lag somit das technische Problem zugrunde, ein in-vitro Verfahren bereitzustellen, das geeignet ist, den Glycosylierungsgrad eines Glycoproteins so einfach und zuverlässig zu bestimmen, daß das Verfahren geeignet ist, die bekannten in-vivo Verfahren z.B. zur Bestimmung von Bioverfügbarkeit und Chargenkonsistenz zu ersetzen.

Die Lösung dieses technischen Problems erfolgt durch die Bereitstellung der in den Patentansprüchen gekennzeichneten Ausführungsformen.

Es wurde überraschenderweise festgestellt, daß Z hervorragend und gut reproduzierbar mit der in in-vivo Verfahren gefundenen Bioverfügbarkeit/ biologischen Aktivität eines Glycoproteins korreliert.
Wegen der guten Reproduzierbarkeit und der analytischen Genauigkeit kann Z vorteilhafterweise auch in einem Verfahren zur Bestimmung der Chargenkonsistenz eingesetzt werden.

Die vorliegenden Untersuchungen lassen den Schluß zu, daß durch Z der "Glycosylierungs-Status" charakterisiert wird. Daher kann mittels der Bestimmung von Z die Glycosylierung auf einfachem Wege verglichen werden.

Unter dem "Glycosylierungs-Status" eines Glycoproteins versteht man im Zusammenhang mit der vorliegenden Erfindung die Zusammensetzung des Glycan-Pools aus bi-, tri- und tetraantennären Glycanen und ihrem jeweiligen Sialylierungsgrad (dem Gehalt an gebundener N-Acetylneuraminsäure) sowie dem Gehalt an Sulfat- oder Phosphat-Gruppen.

Unter der Bioverfügbarkeit eines Glycoprotein-Therapeutikums versteht man die Fähigkeit des Therapeutikums, seine biologische Aktivität bzw. therapeutische Wirksamkeit in vivo zu entfalten. Demnach werden die Bioverfügbarkeit und die biologische Aktivität maßgeblich vom in vivo-Clearance-Verhalten, also der Entfernung des Therapeutikums aus der Blutzirkulation bestimmt. Beispielsweise ist für EPO bekannt, daß es bei einem Fehlen der in den N-glycosidischen Zuckerketten endständig gebundenen N-Acetylneuraminsäure sehr schnell über den sogenannten "asialo-Rezeptor" in der Leber aus der Blutzirkulation entfernt wird und somit seine biologische Wirksamkeit nicht entfalten kann.

Die Z eines therapeutischen Glycoproteins korreliert überraschender Weise mit der in vivo-Halbwertszeit des Glycoproteins und stellt somit einen völlig neuen Meßparameter dar, der es erlaubt, das für das therapeutische Glycoprotein von Charge zu Charge zu erwartende Clearance-Verhalten auf sehr einfache Art und Weise im voraus abzuschätzen. Infolgedessen ermöglicht Z auch eine Aussage über die für das Glycoprotein von Charge zu Charge zu erwartende biologische Sicherheit und seine therapeutische Wirksamkeit. Daher kann man bei Ermittlung von Z eines therapeutischen Glycoproteins von Charge zu Charge z. B. auf die sehr aufwendige, zeitraubende, teure und recht ungenaue Ermittlung der therapeutischen Wirksamkeit des Glycoproteins in Tierexperimenten (in-vivo Assay) verzichten. Dies ermöglicht zudem einen neuen und erheblichen Beitrag zur Reduktion von Tierexperimenten und somit zu einem verbesserten Tierschutz. Gleichzeitig stellt die Z ein besonders geeignetes Maß für die Chargenkonsistenz dar.

Überraschend und von besonderem Wert ist auch die Tatsache, daß man bei endogenen Glycoproteinen, also z. B. bei einem humanem Serumglycoprotein, bei welchem die Glycosylierung etwa mit einer Erkrankung variiert, die Z als einen diagnostischen Meßparameter definieren kann, der mit der Krankheit korreliert und der somit eine Aussage über die Schwere der Erkrankung zuläßt. Dies gilt etwa bei Entzündungserkrankungen, beispielsweise für die "akute Phase Glycoproteine", wie z. B. $\alpha$1-saures Glycoprotein, von welchem bekannt ist, daß sich die Glycosylierung mit dem Auftreten einer Entzündung ändert (De Graaf et al. (1993) *J. Exp. Med.* **177,** 657-666). Dies ist ebenfalls der Fall bei Tumorerkrankungen, bei welchen sich der Gehalt an Protein-gebundener Sialinsäure (und somit die Glycosylierung) mit dem Fortschreiten der Tumorerkrankung verändert (Shahangian et al. (1991) *Clin. Chem.* **37**, 200-204).

Beispielsweise läßt sich durch die Bestimmung der Z eines tumorassoziierten Glycoproteins eines Tumorpatienten eine Aussage über das Stadium der Tumor-Erkrankung machen, ausgehend von der Feststellung, daß sich während des Tumorwachstums die Sialylierung ändert (Shahangian et al., ibid., S. 200).

Kürzlich wurden in einem Sonderband des Glycokonjugate J. (Band 12, Nr. 3, Juni 1995) eine Reihe von Artikeln publiziert, die belegen, daß sich die Glycosylierung bestimmter Glycoproteine mit einer Erkrankung ändert. So eignet sich z. B.

$\alpha$-Fetoprotein für die Früherkennung von Hepatocarcinoma (Aoyagi, ibid., S. 194-199), Blutgruppen-verwandte zirkulierende Kohlenhydrat-Antigene als Tumormarker ($\varnothing$rntoft und Bech, ibid., S. 200-205), $\alpha_1$-Proteinase-Inhibitor und Haptoglobin zur Diagnose von Ovarial-Carcinoma (Turner et al., ibid., S. 211-218), Transferrin für die Charakterisierung von Cerebrospinalflüssigkeit, zur Diagnose von heimlichem Alkoholmißbrauch und des "carbohydrate deficient glycoproteine syndrome" (De Jong et al., ibid., S. 219-226) sowie Glycoformen des $\alpha_1$-sauren Glycoproteins für die Diagnose von Entzündungen und Krebs (Mackiewicz & Mackiewicz, ibid., S. 241-247).

Bei all diesen und weiteren Diagnosen kann Z vorteilhafterweise zur Bestimmung des Erkrankungsstadiums eines Patienten herangezogen werden.

Wesentlich für die Erfindung ist die Erkenntnis, daß die Verteilung der ladungsmäßig einheitlichen Glycangruppen, insbesondere solcher unterschiedlichen Sialylierungsgrades (asialo bis pentasialo), ein entscheidendes Merkmal für die Bioverfügbarkeit eines Glycoproteins und die Chargenkonsistenz ist. Es ist dabei erfindungswesentlich, daß die ladungsgemäß einheitlichen Glycangruppen entsprechend ihrer Ladung, insbesondere ihres Sialysierungsgrades, gewichtet werden. Zusammengefaßt werden diese gewichteten Anteile in der Z.

Die Z eines Glycoproteins läßt sich sehr gut und genau bestimmen, z. B. durch die Verwendung eines optimierten und standardisierten chromatographischen Verfahrens, wie es kürzlich beschrieben wurde (Hermentin et al. (1992) *Anal. Biochem.,* **203,** 281-289).

Die Z eines Glycoproteins wird dadurch bestimmt, daß man

a) den Glycan-Pool des Glycoproteins in an sich bekannter Weise entweder auf chemischem Wege z. B. (mittels Hydrazinolyse) oder auf enzymatischem Wege (z. B. mittels PNGase F) freisetzt und isoliert,
b) denselben in an sich bekannter Weise mittels Ionenaustausch-Chromatographie (bevorzugt mittels HPAE-PAD) primär nach Ladung auftrennt,
c) die prozentualen Flächenanteile der ladungsmäßig getrennten Peak- bzw. Glycangruppen in an sich bekannter Weise bestimmt,
d) die prozentualen Flächenanteile der Peak- bzw. Glycangruppen im neutralen (asialo-, as), monosialo-(MS), disialo- (DiS), trisialo- (TriS), tetrasialo-(TetraS) und pentasialo (PentaS)-Bereich mit null (asialo) bzw. 1 (MS) bzw. 2 (DiS) bzw. 3 (TriS) bzw. 4 (TetraS) bzw. 5 (PentaS) multipliziert und
e) über die jeweils erhaltenen Produkte aufsummiert.

Im Hinblick auf die Bestimmung von Z können die Asn-verknüpften Zuckerketten der Glycoproteine in an sich bekannter Weise prinzipiell auf zwei Arten freigesetzt werden - nämlich entweder auf chemischem Wege (z. B. mittels Hydrazinolyse) oder enzymatisch (z. B. mittels N-Glycanase bzw. PNGase F). Das enzymatische Verfahren erfordert von Fall zu Fall zu optimierende Reaktionsbedingungen - etwa einen voranzustellenden tryptischen Verdau des Glycoproteins oder den Zusatz eines geeigneten Detergenz. Und auch die Hydrazinolyse erfordert spezielles Know-how, wenn man die Nebenreaktionen minimieren möchte. Sie kann jedoch heute mit einem Gerät der Fa. Oxford GlycoSystems (dem GlycoPrep 1000) vollautomatisch durchgeführt werden.

In der HPAEC werden die N-Glycane primär nach Ladung, d. h., nach der Anzahl ihrer Sialinsäure-Reste aufgetrennt (Hermentin et al. (1992) *Anal. Biochem.* **203**, 281-289), weshalb sie sich für die Bestimmung der Z von Glycoproteinen in vorzüglicher Weise eignet.

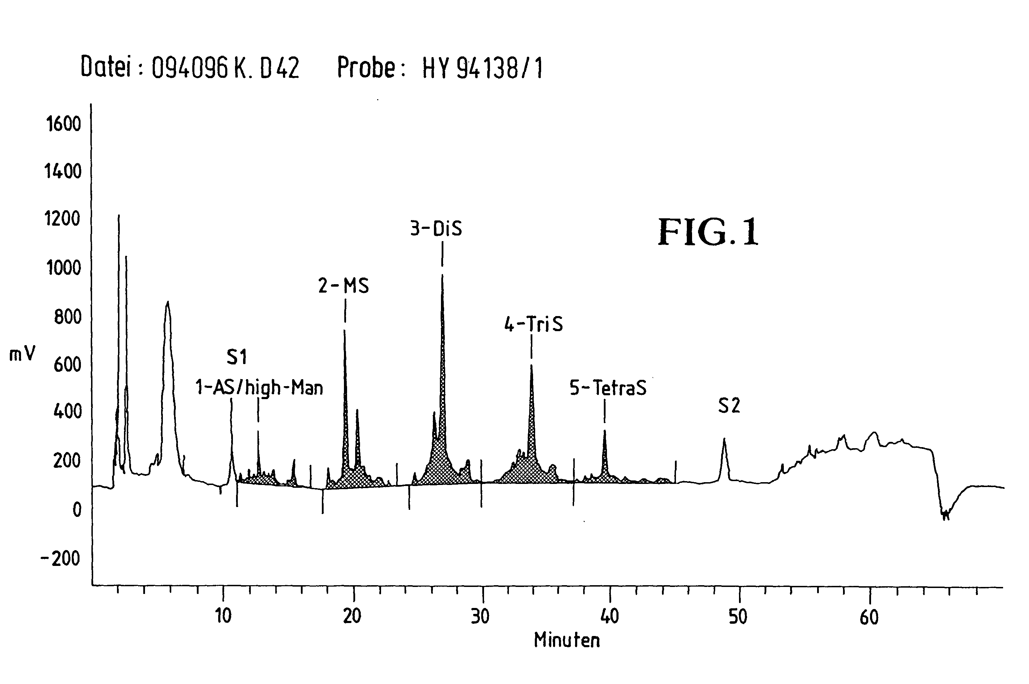

So wurde gefunden, daß sich beim Mapping der N-Glycan-Pools von Glycoproteinen mittels HPAE-PAD die Glycane gleicher Ladung - das sind in der Regel Glycane mit der gleichen Anzahl an Neu5Ac-Resten - meist in deutlich voneinander getrennte Peakgruppen zusammenfassen lassen. Dies wird am Beispiel des Glycan-Pools von rhu IL-4R (CHO) beispielhaft dargestellt (**Figur 1**). Bewährt hat sich dabei die Verwendung zweier internaler Standards, von denen der eine (S1 = z. B. LNnT oder LNFP-V, Oxford GlycoSystems) vor den einzelnen Peaks des Glycan Pools und der andere [S2 = (Neu5Ac)3] nach den einzelnen Peaks des Glycan-Pools eluiert, so daß sich die detektierten Glycane stets im RT-Bereich zwischen den beiden Standards befinden und sich ihre Gesamtpeakfläche, die 100% entspricht, mit Hilfe der Chromatographie-Auswertesoftware leicht ermitteln läßt. Die Gesamtpeakfläche erhält man somit durch Integration und Summation der Peaks, die sich zwischen den Retentionszeiten der beiden Standards S1 und S2 befinden. Auf demselben Wege lassen sich auch die ladungsmäßig getrennten Peakgruppen mit 0, 1, 2, 3, 4 und 5 negativen Ladungen integrativ zusammenfassen und ihre jeweilige Peakgruppenfläche "F" als prozentualen Anteil der Gesamtpeakfläche des Glycan-Pools errechnen. Dabei wir für sämtliche Glycane der gleiche Responsefaktor angenommen.

Die Berechnung der hypothetischen Ladungszahl Z erfolgt nach Gleichung 1:

$$Z = F_{(as)} * 0 + F_{(MS)} * 1 + F_{(DiS)} * 2 + F_{(TriS)} * 3 + F_{(TetraS)} * 4 + F_{(PentaS)} * 5 \tag{I}$$

wobei $F_{(as)}$, $F_{(MS)}$, $F_{(DiS)}$, $F_{(TriS)}$, $F_{(TetraS)}$ und $F_{(PentaS)}$ jeweils den prozentualen Anteil der Peakgruppenfläche im asialo-, monosialo-, disialo-, trisialo-, tetrasialo- bzw. pentasialo-Bereich darstellt, bezogen auf die Peakflächen = 100%.

Auf diese Weise erhält man für ein Glycoprotein, welches überwiegend tetraantennäre tetrasialo- (C4-4*)-Strukturen aufweist, wie z. B. rekombinantes Erythropoietin, eine Z von etwa 400. Analog erhält man für ein Glycoprotein, welches überwiegend triantennäre trisialo- (C3-3*)-Strukturen aufweist, wie z. B. bovines Fetuin, eine Z von etwa 300 und für ein Glycoprotein, welches überwiegend biantennäre disialo- (C2-2*)-Strukturen aufweist, wie z. B. humanes Antithrombin III, eine Z von etwa 200. Für ein Glycoprotein, welches beispielsweise nur asialo-Strukturen aufweist, wie z. B. Rinder-Pankreas-Ribonuclease B, oder sogenannte "trunkated forms" (Rumpfstrukturen) aufweist, wie z. B. Hühner-Ovomucoid, erhält man eine Z von etwa 0.

In analytischer Hinsicht ist es wünschenswert, den aus einem Glycoprotein isolierten Glycan-Pool zunächst chromatographisch nach dem Grad der Sialylierung aufzutrennen, bevor dann je nach Bedarf weitere Trennmethoden zum Einsatz kommen. Dies kann mittels Anionen-Austauschersäulen erfolgen, wie etwa Glycopac™ DEAE (Waters), Mono Q™ (Pharmacia), Gen-Pak™ FAX (Waters) oder mittels der HPAEC (high-pH anion-exchange chromatography) an pellicularen Anionenaustauscher-Harzen (CarboPak PA-1™ oder CarboPak PA-100™, Dionex) (Lee und Rice (1994) in: "Glycobiology - A Practical Approach", Kapitel 3C, S. 127-163, IRL Press, Herausgeber: Fukuda und Kobata).

Kürzlich wurde gezeigt, daß die "high-pH anion-exchange chromatography with pulsed amperometric detection" (HPAE-PAD) die Kriterien für eine schnelle, kostengünstige und vor allem gut reproduzierbare chromatographische Methode zur strukturellen Zuordnung der Oligosaccharid-Ketten von Glycoproteinen in vorzüglicher Weise erfüllt (Hermentin et al. (1992) *Anal. Biochem.* **203,** 281-289; Hermentin et al. (1992) *Anal. Biochem.* **206,** 419-429). Bei diesem Verfahren erfolgt die Trennung der Oligosaccharide unter alkalischen Bedingungen an einer Anionenaustauscher-Säule (CarboPac PA-1 oder CarboPac PA-100 der Fa. Dionex). Dieses Verfahren trennt die vom Glycoprotein isolierten N-Glycane zunächst nach ihrer Ladung und ermöglicht so eine Aussage über die Zusammensetzung des N-Glycan-Pools aus neutralen (asialo) sowie mono-, di-, tri- und tetrasialo-Stukturen (also N-Glycanen mit null bis vier negativ geladenen Neuraminsäure-Resten). Die Vermessung der Zucker erfolgt sehr selektiv und sensitiv - und ohne Derivatisierung der Glycane - durch gepulste amperometrische Detektion an einer Goldelektrode.

Die Bestimmung von Z wurde in zahlreichen Experimenten mit rhu IL-4R (einem therapeutischen Glycoprotein der Behringwerke AG) beispielhaft validiert. Dabei konnte gezeigt werden, daß die hypothetische Ladungszahl als ein neuer, aussagekräftiger, verläßlicher und charakteristischer Parameter für die Protein-Glycosylierung angesehen werden kann.

Im folgenden finden sich verschiedene Beispiele für die Bestimmung von Z aus den erhaltenen HPAE-PAD-Chromatogrammen. Hierzu wurden die Glycane aus verschiedenen Glycoproteinen in an sich bekannter Weise entweder mittels automatisierter Hydrazinolyse (unter Verwendung des GlycoPrep 1000™, Oxford GlycoSystems, OGS) oder auf enzymatischem Wege (mittels PNGase F) freigesetzt und isoliert oder von der Firma Oxford GlycoSystems direkt als Glycan Pools eingekauft und mittels HPAE-PAD im Standardgradienten "S" (Hermentin et al. (1992) *Anal. Biochem.* **203,** 281-289) vermessen.

Die Bestimmung der Z ist am Beispiel des HPAE-PAD Chromatograms von rhu IL-4R (**Figur 1**) beispielhaft aufgezeigt; die zugehörige Berechnung findet sich in **Tabelle 1**. Für alle anderen Beispiele erfolgte die Bestimmung von Z analog.

Die Bestimmung der Z für α1-saures Glycoprotein (AGP) weicht von der Bestimmung der anderen genannten Glycoproteinen etwas ab, weil AGP in seinen N-Glycanen antennäre Fucose-Reste aufweist (Hermentin et al. (1992) *Anal. Biochem.* **206,** 419-429). Derartige N-Glycane eluieren in der HPAEC im Standard-Gradienten "S" im Vergleich zum korrespondierenden nicht-fucosylierten N-Glycan um etwa 4 min früher. Deshalb kommt es im Falle des N-Glycan Pools von AGP nicht zu der in den anderen Beispielen beobachteten sauberen Trennung in ladungsmäßig einheitliche Peakgruppen, sondern zu einer Überlagerung dieser Peakgruppen (Hermentin et al. (1992) *Anal. Biochem.* **206,** 419-429). Die Bestimmung der hypothetischen Ladungszahl erfolgte im Falle von AGP in Anlehnung an die ladungsmäßige Peak-Zuordnung nach Hermentin et al. (1992) (*Anal. Biochem.* **206,** 419-429). Auf diese Weise wurde die Z in den Beispielen 9 und 10 bestimmt.

In den Fällen, wo die N-Glycane eines Glycoproteins neben Neuraminsäure z. B. auch Sulfatgruppen enthalten, erfolgt die Bestimmung der Z z. B. so, daß die ladungsmäßig getrennten Peakgruppen, die im Chromatogramm im Bereich von 6 oder 7 Ladungen liegen, mit 6 bzw. 7 multipliziert werden.

Die für eine Reihe von Glykoproteinen bestimmte Z sind in Tabelle 3 zusammengefaßt.

Die nachstehend genannten Beispiele schränken die Erfindung nicht ein.


**Beispiel 1:**

**Validierung der Bestimmung von Z mit rhu IL-4R (CHO) (Charge E4-930914)**

a) Validierung mit 1.0 mg rhu IL4R pro Hydrazinolyse-Reaktor

Die Bestimmung von Z wurde mit rhu IL-4R (CHO) als Referenz-Glycoprotein validiert. Hierzu wurde der Glycan-Pool der IL-4R-Probe (Charge E4-930914, Behringwerke AG) an 3 verschiedenen Tagen in 6 verschiedenen Ansätzen mittels automatischer Hydrazinolyse - in Gegenwart von LNFP-V als internalem Standard S1 - (Glyco-Prep 1000™, Oxford GlycoSystems; gleichzeitige Hydrazinolyse an beiden Reaktoren; Ansatz jeweils 1 mg IL-4R pro Reaktor) freigesetzt und isoliert. Jeder der 6 Glycan-Pools wurde über Sephadex G-25 superfein (Pharmacia) (Säulenfüllung 21 x 1 cm) entsalzt und dreimal (an drei verschiedenen Tagen; jeweils unter Zusatz von S2 = (Neu5Ac)3) in der HPAE-PAD vermessen, und aus dem jeweils erhaltenen Chromatogramm wurde anhand von Gleichung 1 jeweils Z bestimmt. In Tabelle 1 sind die jeweils integrierten Peakflächenanteile für jeden der 18 HPAE-PAD-Einzelläufe sowie die jeweils durchgeführte Summation gemäß Gleichung 1 detailliert aufgeführt. Als illustratives Beispiel und Referenz-Lauf dient das HPAE-PAD-Mapping-Chromatogramm aus **Figur 1.**

Dabei wurde Z für die genannte IL-4R-Probe mit **Z = 201 +/- 3 (VK = 1.4 %)** mit sehr hoher Reproduzierbarkeit bestimmt **(Tabelle 2).**

b) Validierung mit 0.50 mg rhu IL-4R pro Hydrazinolyse-Reaktor

In einem zweiten Validierungs-Experiment wurde die Hydrazinolyse in 6 verschiedenen Ansätzen analog Beispiel 1a) mit 0.5 mg rhu IL-4R pro Reaktor durchgeführt und gemittelt. Dabei wurde die Z mit **Z = 194+/-5 (VK = 2.3 %)** bestimmt **(Tabelle 2).**

Tabelle 1: Berechnung von Z des Mapping Profils von rhu IL-4R (CHO) (Charge E4-9300914) aus Figur 1

| File Nr. | Peak-gruppe Nr. | Retentionszeit hoechster Peak (min) | Integrationsbereich (Peakgruppe) | prozentualer Anteil der Peakgruppen-fläche (%) | Multiplikator | Ladungszahl-Anteil |
|---|---|---|---|---|---|---|
| 0940964K.D42 | 1 | 12,57 | asialo/high Mannose | 9,70 | 0 | 0 |
| | 2 | 19,23 | monosialo (MS) | 23,31 | 1 | 23,31 |
| | 3 | 29,69 | disialo (DiS) | 31,81 | 2 | 63,62 |
| | 4 | 33,73 | trisialo (TriS) | 25,38 | 3 | 76,14 |
| | 5 | 39,44 | tetrasialo (TetraS) | 9,81 | 4 | 39,24 |
| | | | Gesamtpeakfläche | 100,01 | | Ladungszahl 202 |

**Beispiel 2:**

**Bestimmung der Z von rhu IL-4R (CHO) (Charge E4-930914) nach PNGase F-Verdau - ohne und mit CHAPS**

Die Freisetzung der N-Glycane erfolgte nach Reduktion des Glycoproteins (500 μg) mittels Dithioerythrol (DTE; 25 μl einer 0.3 M wäßrigen DTE-Lösung) während 10 min bei 70°C. Der Überschuß an DTE wurde durch Ankonzentrierung in einer Centricon-Kartusche mit Ausschlußgrenze 10.000 D (Fa. Amicon) entfernt, und das Konzentrat wurde dreimal mit Glycanase-Verdaupuffer im Centricon-Röhrchen nachgewaschen. Dann wurde das Konzentrat in ein Eppendorf-Hütchen überführt und in Glycanase-Verdaupuffer (500 μl) a) mit und b) ohne die Gegenwart von 0.5 % CHAPS mittels PNGase F (Boehringer Mannheim; 5 units) in 50 mM Natriumphosphat-Puffer, pH 7.6 für 48 h bei 37 °C verdaut.

Nach Entsalzung analog Beispiel 1 a wurden folgende Z bestimmt:

a) (ohne CHAPS): **Z = 208;**

b) (mit CHAPS): **Z = 206 (Tabelle 2).**

Somit konnte gezeigt werden, daß durch den Zusatz des Detergenz CHAPS keine Verbesserung der Deglycosylierung erzielt werden kann.

c) Alternativ wurde rhu IL-4R nach Reduktion mittels Dithiothreitol (DDT) carboxamidomethyliert und mittels Trypsin verdaut, wie bei Hermentin et al. (Anal. Biochem. (1992), **206** , 419) beschrieben. Danach erfolgte die Glycan-Freisetzung analog Beispiel 2 b) in Gegenwart von 0.5 % CHAPS. Dabei wurde die Z zu Z = 200 bestimmt (**Tabelle 2**).

d) Analog Beispiel 2 c) erfolgte der Verdau des rhu IL-4R mittels des Enzyms Lys C anstelle des Enzyms Trypsin.

Ansonsten wurde wie in Beispiel 2 c) verfahren. Dabei wurde die Z zu Z = 204 bestimmt **(Tabelle 2)**.
Der Mittelwert aus den gemäß den Beispielen 2 a) - 2 d) bestimmten Ladungszahlen beträgt Z = 204,5 ± 3.4 (VK = 1.7 %) **(Tabelle 2).**

Diese vier Ladungszahlen stehen in guter Übereinstimmung mit der unter den Beispielen 1a und 1b nach automatischer Hydrazinolyse bestimmten mittleren Ladungszahl von Z = 201 (1a; 1.0 mg IL-4R pro Reaktor) bzw. Z = 194 (1b; 0.5 mg IL-4R pro Reaktor). **(Tabelle 2)**.

**Tabelle 2**
**Validierung der Bestimmung der Z mittels rhu IL-4R (CHO) als Modellglycoprotein**

| Charge | Herstellung des Glycan-Pools | Z Einzel-bestimmung | Z Mittel-wert | Stdabw | VK | Anzahl an Experi-menten | Anzahl an HPAE-PAD-Läufen | Beisp. |
|---|---|---|---|---|---|---|---|---|
| E4-930914 | GlycoPrep (1.0 mg/Reaktor) | | **201** | +/-3 | 1.4% | 6 | 18 | 1a |
| E4-930914 | GlycoPrep (0.5 mg/Reaktor) | | **194** | +/-5 | 2.3% | 6 | 18 | 1b |
| E4-930914 | PNGase F (ohne CHAPS) | 208 | | | | | | 2a |
| E4-930914 | PNGase F (mit 0.5% CHAPS) | 206 | | | | | | 2b |
| E4-930914 | PNGase F (Trypsin/mit 0.5% CHAPS) | 200 | **204,5** | +/-3.4 | 1.7% | 4 | 4 | 2c |
| E4-930914 | PNGase F (Lys C/mit 0.5% CHAPS) | 204 | | | | | | 2d |
| B11-930406 | GlycoPrep (0.5 mg/Reaktor) | | **243** | | | | | 3 |
| B11-930406 | PNGase F (ohne CHAPS) | 241 | | | | | | 4a |
| B11-930406 | PNGase F (mit 0.5% CHAPS) | 246 | **243,5** | +/-3.5 | 1.5% | 2 | 2 | 4b |

EP 0 843 821 B1

**Beispiel 3:**

**Bestimmung der Z von rhu IL-4R (CHO) (Charge B11 -930406) nach automatischer Hydrazinolyse**

Die Hydrazinolyse erfolgte analog Beispiel 1b mit 0.5 mg rhu IL-4R. Für die Z wurde **Z = 243** bestimmt.

**Beispiel 4:**

**Bestimmung der Z von rhu IL-4R (CHO) (Charge B11-930406) nach PNGase F-Verdau - ohne und mit CHAPS**

Die Freisetzung der N-Glycane erfolgte analog Beispiel 2 a) bzw. 2 b).

Es wurden folgende Z bestimmt: a) (ohne CHAPS) **Z = 241;**
b) (mit 0.5% CHAPS) **Z = 246;** Mittelwert **Z = 243.5 ± 3.5** (1.5 %) **(Tabelle 2).**

Diese beiden Z stehen in guter Übereinstimmung mit der unter Beispiel 3a nach Hydrazinolyse bestimmten Ladungszahl von **Z = 243,** so daß für die Charge **B11-930406** von rhu IL-4R ein Z von **Z = 243** angegeben werden kann **(Tabelle 2).**

Somit wurde gefunden, daß das Material aus Klon **B11-930406 (Z = 243)** (Beispiele 3 und 4) gegenüber dem Material aus Klon **E4-930914 (Z = 204.5)** (Beispiele 1 und 2) - bei gleichen Kultivierungs-, Ernte- und Aufreinigungsbedingungen - einen größeren Anteil an höher geladenen bzw. höher-antennären Glycanen aufweist.

**Beispiel 5:**

**Ein Vergleich von Z mit an sich bekannten Glycoanalytik-Parametern sowie dem in vivo-Clearance-Verhalten eines Glycoproteins im Mausmodell**

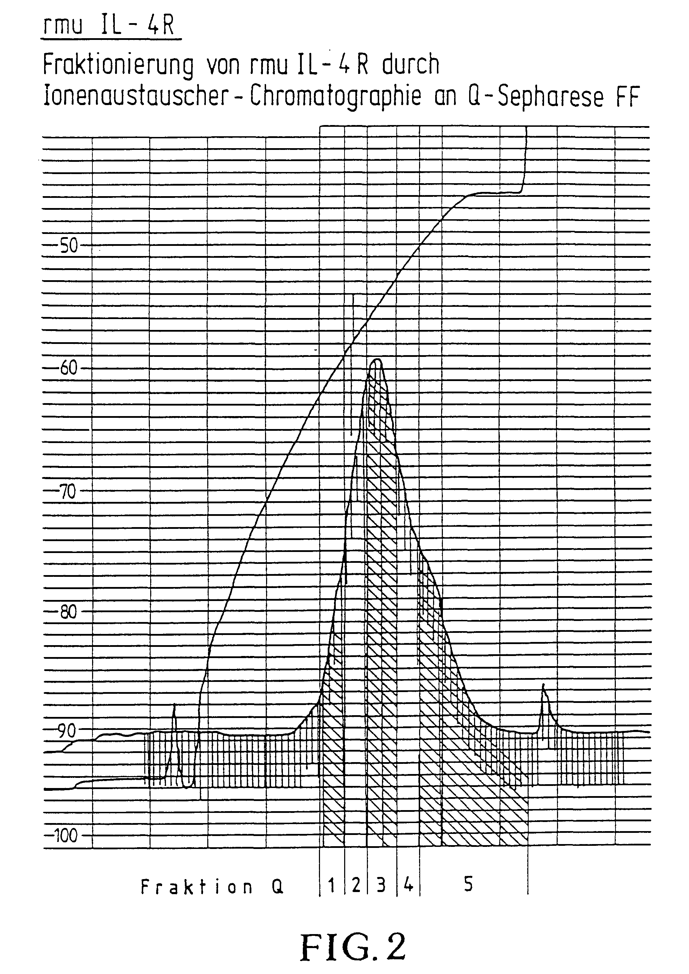

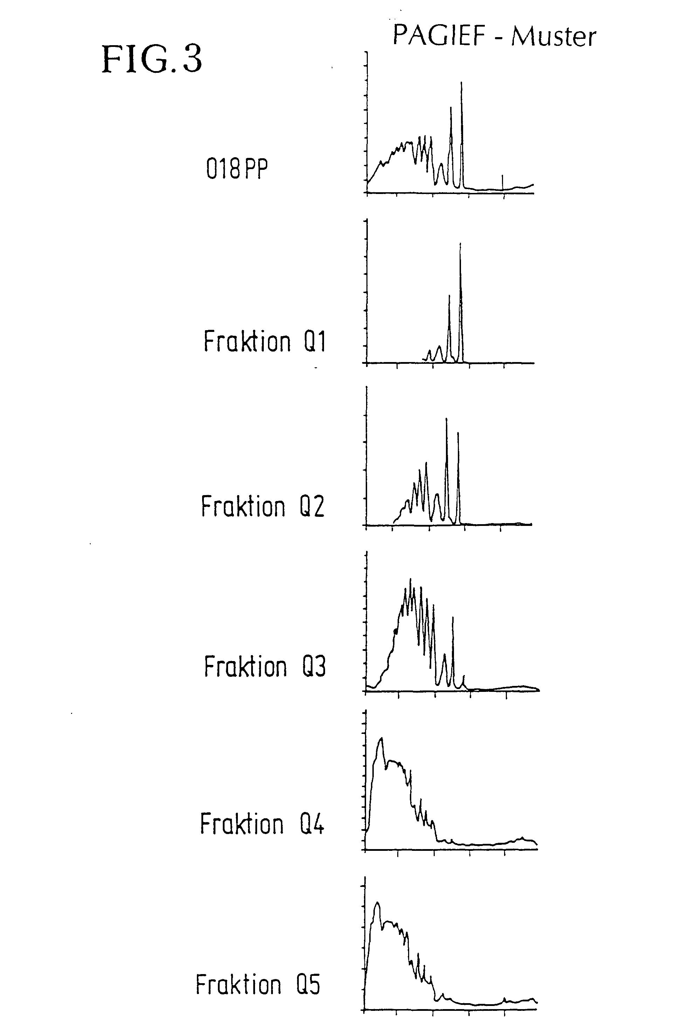

Eine Präparation von aus BHK-Zellen gewonnenem löslichem murinem Interleukin-4-Rezeptor (rmur IL-4R, Behringwerke AG) wurde in an sich bekannter Weise über ein Anionenaustauscher-Harz (Q-Sepharose, Pharmacia) in 5 Fraktionen Q1-Q5 aufgetrennt (**Figur 2**). Die Analytik der Einzelfraktionen (isoelektrische Focussierung, Sialinsäure-Bestimmung, Gehalt an Monosaccharid-Komponenten) wurde in an sich bekannter Weise durchgeführt. Das IEF-Bandenmuster wurde in an sich bekannter Weise mittels einer Gel-Auswertesoftware gescanned. Die erhaltenen Banden-Scans sind in **Figur 3** dargestellt. Die in an sich bekannter Weise gewonnenen analytischen Daten sind in **Tabelle 3** ausgewiesen.

Die Bestimmung des Gehalts an N-Acetylneuraminsäure (Neu5Ac; Sialinsäure) erfolgte nach Hermentin und Seidat (in: *GBF Monographs,* Vol 15, pp 185-188, H. S. Conradt, ed., VCH, Weinheim / New York / Cambridge)

Die Monosacchrid-Bestimmung erfolgte in an sich bekannter Weise nach Hardy et al. *(Anal. Biochem.* (1988) **170,** 54-62). Aus dem Ergebnis der Monosaccharid-Analytik wurde der Quotienten Neu5Ac/Gal (mol/mol) bestimmt.

Anmerkung:
Da Neu5Ac bei normalen N-Glycanen stets an antennäre Galactose-Reste gebunden ist, erlaubt der Quotient aus Neu5Ac/Gal (mol/mol) eine Berechnung des Gehalts an terminaler Galactose. Die terminale Galactose der N-Glycane wiederum beeinflußt das Clearance-Verhalten eines Glycoproteins, da Glycoproteine mit terminaler Galactose in den N-Glycanen über den sogenannten asialo-Rezeptor in der Leber aus dem Blut eliminiert werden. Der Sialylierungsgrad (Neu5Ac/Gal; mol/mol) erlaubt somit eine Aussage über die zu erwartende Clearance eines Glycoproteins in der Leber. Die Bestimmung des Sialylierungsgrads gestaltet sich jedoch als relativ ungenau, da sich im Sialylierungsgrad die Varianzen beider Tests (der Neu5Ac- und der Gal-Bestimmung) addieren. Daher ist ein genauerer Parameter notwendig, der das Clearance-Verhalten eines Glycoproteins bei in vivo-Applikation beschreibt.

Aus dem Ergebnis der Monosaccharid-Analytik wurde ferner der Quotient aus dem molaren Anteil an Mannose und Galactose (Man/Gal; mol/mol) ermittelt, der einen Hinweis auf die Zusammensetzung der N-Glycane aus "high-mannose"-Strukturen und "complex-type"-Strukturen erlaubt.

Anmerkung:
Das Man/Gal-Verhältnis erlaubt ebenfalls eine Aussage über das zu erwartende Clearance-Verhalten eines Glycoproteins, da Glycoproteine mit "high mannose"-Strukturen ebenfalls über einen Rezeptor in der Leber (den sogen. "high mannose"-Rezeptor) aus der Blutzirkulation entfernt werden. Da der Gehalt an "high-mannose"-Strukturen in die Berechnung der hypothetischen Ladungszahl gemäß Gleichung 1 mit eingeht, reflektiert Z auch den Gehalt an "high-Man-Strukturen" und erlaubt somit auch eine Vorhersage der Clearance über den "high-Man-Rezeptor".

Die Z wurde für die Einzelfraktionen analog Beispiel 1 bestimmt.

Tabelle 3:

| | Neu5Ac (ug/ mg) | NeuAc/Ga (mol/ mol) | Man/Gal (mol/ mol) | AUD (ug/ ml*min) | CL (ml/ min) | Z |
|---|---|---|---|---|---|---|
| **Ausgang:** | 78.5 | 0,54 | 1,28 | 42.7 | 0,23 | n.b. |
| Fraktion Q1 | 13.6 | 0,20 | 3,02 | 0.8 | 10,0 | 147 |
| Fraktion Q2 | 38.2 | 0,42 | 1,99 | 5.3 | 2,00 | 191 |
| Fraktion Q3 | 82.9 | 0,62 | 1,24 | 33.6 | 0,29 | 238 |
| Fraktion Q4 | 109.5 | 0,67 | 1.04 | 63.7 | 0,16 | 248 |
| Fraktion Q5 | 108.6 | 0,63 | 0,98 | 85.9 | 0,12 | 247 |

Desweiteren wurden die Einzelfraktionen in an sich bekannter Weise auf ihr Clearance-Verhalten im Mausmodell (AUD) überprüft **(Tabelle 3)**.

Hierzu wurden Aliquote der Einzelfraktionen in Mäuse injiziert und die Clearance von rmur IL-4R aus dem Mäuseblut im ELISA verfolgt.

Die Clearance-Rate von IL-4R wurde wie folgt bestimmt:

Weibliche BALB/C-Mäuse erhielten in die Schwanzvene eine Bolus-Injection von IL-4R (10 µg 0.2 µg/kg). Fünf, 10 und 30 min. nach Applikation wurden mittels Punktion des retroorbitalen Venenkomplexes Blutproben zur Serumgewinnung entnommen. Die IL-4R-Konzentration in der jeweiligen Serumprobe wurde mittels eines ELISA bestimmt. Die Kurve der im Serum bestimmten IL-4R-Konzentration gegen die Zeit ($AUD_{5\text{-}30}$) wurde unter Anwendung der sogenannten Trapez-Regel (Koch (1985), *Apoth. Ztg.,* **29,** Folge 17, April 1975,) berechnet. Hieraus wurde die initiale Clearance ($Cl_{5\text{-}30}$) nach der Gleichung $\mathbf{Cl_{5\text{-}30} = Dosis/AUD_{5}\text{-}_{30}}$) berechnet.

Ergebnis:

Wie aus den in **Figur 3** ausgewiesenen Scans der IEF-Banden ersichtlich, ergeben sich in der IEF kontinuierlich verlaufende Unterschiede im Bandenmuster der Glycoformen für die Fraktionen Q1 bis Q4, während sich die Fraktionen Q4 und Q5 in der IEF als nicht unterscheidbar erweisen. Die isolektrische Focussierung birgt jedoch den Nachteil, daß sie quantitativ nicht oder nur sehr schwer faßbar ist und somit in erster Linie nur einen qualitativen Meßparameter abgibt.

Der Sialinsäure-Gehalt nimmt von Fraktion Q1 (13.6 µg/mg) nach Fraktion Q4 (109.5 µg/mg) kontinuierlich zu, während er sich in den Fraktionen Q4 und Q5 als praktisch identisch erweist **(Tabelle 3).** Die Ergebnisse der Sialinsäure-Bestimmung korrelieren daher gut mit den Ergebnissen der isoelektrischen Fokussierung.

Ähnlich wie der Sialinsäure-Gehalt, jedoch weniger ausgeprägt, steigt auch der Sialylierungsgrad (Quotient Neu5Ac/Gal) von Fraktion Q1 (0.20 Neu5Ac-Reste pro Galactose-Rest) nach Fraktion Q4 (0.67 Neu5Ac-Reste pro Galactose-Rest) an, fällt jedoch in Fraktion Q5 (0.63 Neu5Ac-Reste pro Galactose-Rest) wieder in etwa auf den Wert der Fraktion Q3 (0.62 Neu5Ac-Reste pro Galactose-Rest) ab **(Tabelle 3)**. Dieser Abfall des Analysenwerts von Fraktion Q5 auf den Wert der Fraktion Q3 wurde weder bei der Sialisäure-Bestimmung noch bei der isoelektrischen Focussierung beobachtet. Es ist daher zu vermuten, daß der Sialylierungsgrad (Neu5Ac/Gal; mol/mol) einen weniger verläßlichen Parameter darstellt als die Neu5Ac-Bestimmung oder die isoelektrische Focussierung, weil sich im Quotienten des Sialylierungsgrads die Ungenauigkeiten der beiden Einzeltests (also der Neuraminsäure-Bestimmung und Monosaccharid-Komponentenanalytik) addieren.

Umgekehrt nimmt der Quotient Man/Gal von Fraktion Q1 (3.02 mol/mol) nach Fraktion Q4 (1.04 mol/mol) kontinuierlich ab und bleibt in Fraktion Q5 im Rahmen der Meßgenauigkeit praktisch konstant (0.98 mol/mol) **(Tabelle 3).** Der Quotient Man/Gal erscheint wegen seines von Fraktion Q1 nach Q5 kontinuierlichen Verlauf wiederum als ein verlässlicher und aussagefähiger Parameter - ähnlich wie die Neu5Ac-Bestimmung und die isoelektrische Focussierung.

Die gemäß Gleichung 1 bzw. Beispiel 1 bestimmte Z nimmt, wie zu erwarten, parallel zum Sialinsäure-Gehalt zu und verläuft kontinuierlich von Z = 147 (Fraktion Q1) nach Z = 248 (Fraktion Q4), während sie von Fraktion Q4 (Z = 248) nach Fraktion Q5 (Z = 247) konstant bleibt **(Tabelle 3).** Somit spiegelt die Bestimmung von Z sehr gut sowohl die Ergebnisse der Sialinsäure-Bestimmung als auch den qualitativen Verlauf der isoelektrischen Focussierung wieder und erscheint (wie die Neu5Ac-Bestimmung und die IEF) der Bestimmung des Sialylierungsgrads (Neu5Ac/Gal; mol/mol) überlegen. (Daß sich die Bestimmung von Z aber auch gegenüber der Neu5Ac-Bestimmung als überlegen erweist, wird im nachfolgenden Beispiel 6 gezeigt).

Das Clearance-Verhalten (AUD, area under data; µg/ml*min) der Fraktionen Q1-Q5 wurde ebenfalls in an sich bekannter Weise bestimmt. Dabei ist die Zirkulations-Halbwertszeit des IL-4R im Blut der Maus umso größer, je größer die AUD. Wie **Tabelle 3** zu entnehmen, steigt die AUD von Fraktion Q1 (AUD = 1) nach Fraktion Q5 (AUD = 86) kontinuierlich an. Somit ergibt sich eine gute Korrelation der AUD mit der IEF, dem Sialinsäure-Gehalt, dem Quotienten Man/Gal (mol/mol) und der Z für die Fraktionen Q1-Q4. Lediglich für Fraktion Q5 ergibt sich eine Abweichung der AUD von den genannten Meßparametern. Somit wurde gefunden, daß die Clearance eng mit der Ladungszahl korreliert und daß Z - im Vergleich mit einem geeigneten Standard - eine gute Aussage über das zu erwartende Clearance Verhalten eines Glycoproteins bei in vivo-Applikation erlaubt.

Anmerkung:

Ob es sich bei dieser Abweichung der AUD von den restlichen analytischen Meßwerten um einen realen Sachverhalt oder um einen Artefakt (Ausreißer) handelt, konnte mangels Materials nicht reproduziert werden. Ferner ist festzuhalten, daß die AUD aus Tierschutz-Gründen jeweils nur mit einer Maus pro AUD-Meßpunkt ermittelt wurde, was die Verläßlichkeit des AUD-Wertes im Vergleich zu den analytischen Meßwerten relativiert.

Ergebnis:

Die Z besitzt somit für die Vorhersage des zu erwartenden Clearance-Verhaltens eines Glycoproteins in vivo den besonderen Vorteil, daß Z gemäß Gleichung 1 sowohl die zu erwartende Clearance über den asialo-Rezeptor als auch die zu erwartende Clearance über den high-Man-Rezeptor erfaßt und somit eine genauere Vorhersage über des zu erwartenden Clearance-Verhaltens ermöglicht, als jede andere der genannten Analysenmethoden.

**Beispiel 6:**

**Z und die Lagerstabilität von rhu IL-4R (CHO)**

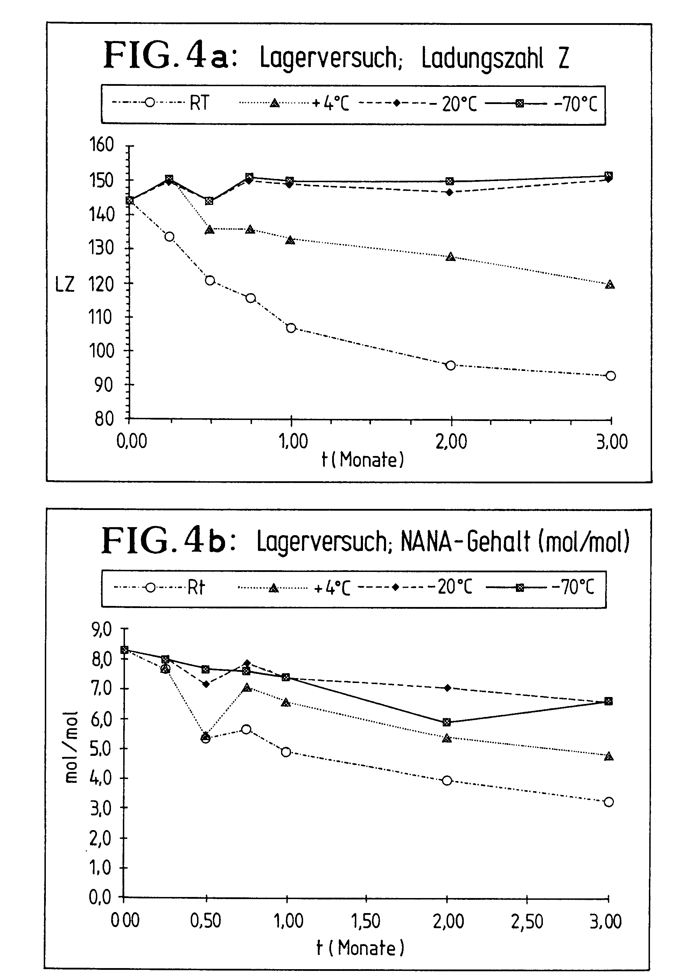

Zur Bestimmung der Lagerungsfähigkeit von IL-4R im Fermenter-Erntemedium wurden Aliquots der Ernten bei verschiedenen Temperaturen (RT, +4°C, -20°C und -70°C) gelagert. Zum Zeitpunkt Null sowie nach 1, 2 und 3 Monaten wurden Aliquots entnommen. Aus diesen Aliquots wurde der IL-4R in an sich bekannter Weise mittels Affinitätschromatographie an einem immobilisierten anti-IL-4R monoklonalen Antikörper aufgereinigt. Von den gereinigten lL-4R-Proben wurde jeweils der Neuraminsäure-Gehalt sowie die Z (analog Beispiel 1) bestimmt. Die Ergebnisse sind in **Figur 4** zusammengefaßt.

Wie aus **Figur 4a** ersichtlich, erwies sich die Z für die bei -70°C und -20°C gelagerten Proben als konstant, fiel jedoch bei den bei +4°C deutlich und bei den bei RT gelagerten Proben mit zunehmender Lagerdauer massiv ab.

Die Ergebnisse der Neuraminsäure-Bestimmung (**Figur 4b**) sind von deutlich geringerem Aussagewert, obwohl sich eine der Ladungszahl analoge Tendenz erkennen läßt. Somit liegt auf der Hand, daß die Bestimmung der Ladungszahl in analytischer Hinsicht der Neuraminsäure-Bestimmung überlegen ist. Der Vorteil gründet in der hohen Genauigkeit, mit welcher sich die Ladungszahl bestimmen läßt - mit dem besonderen Vorteil, daß für die Bestimmung der Ladungszahl - im Gegensatz zur Neuraminsäure-Bestimmung - der Bezug auf die (Glyco)Protein-Konzentration nicht erforderlich ist und somit die Ungenauigkeit der Proteinbestimmung nicht in das Testergebnis eingeht.

Somit konnte gezeigt werden, daß sich Z in hervorragender Weise als Parameter zur Überprüfung der Lagerungsstabilität eines Glycoproteins eignet.

**Beispiel 7:**

**Bestimmung der Z von rhu EPO (BHK)**

Die Freisetzung der N-Glycane erfogte aus rhu EPO (BHK) (Merckle AG) mittels PNGase F in an sich bekannter Weise (Nimtz et al., *Eur. J. Biochem.* (1993) **213,** 39-56).

Die Z wurde analog Beispiel 1 mit **Z = 323** bestimmt **(Tabelle 4)**.

Anmerkung:

Von Nimtz et al. (*Eur. J. Biochem.* (1993) **213,** 39-56) wurde die Glycosylierung von rhu EPO (BHK) (Merckle AG) in einer detaillierten analytischen Studie (mittels GC-MS, FAB-MS und $^1$H-NMR) sehr ausführlich untersucht. Demnach enthält der N-Glycan Pool des besagten rhu EPO (BHK) 40.9% tetrasialylierte, 35.0% trisialylierte und 21.1% disialylierte N-Glycane. Aus diesen Angaben errechnet sich für den N-Glycan-Pool unter Verwendung von Gleichung 1 eine Z von Z = 40.9x4 + 35.0x3 + 21.1x2 = 311. Überraschenderweise steht diese nach den Angaben von Nimtz et al. errechnete Ladungszahl von Z = 311 in sehr guter Übereinstimmung mit der gemäß Beispiel 1 errechneten Ladungszahl von Z = 323. Der Unterschied beider Ladungszahlen beträgt 12, was einer prozentualen Differenz von weniger als 4% entspricht. Die Bestimmung der Ladungszahl nach Beispiel 1 gestaltet sich jedoch vergleichsweise sehr viel billiger, schneller und einfacher. Somit erweist sich Z als ein neuer und sehr hilfreicher und vorteilhafter Meßparameter für die Charakterisierung des Glycosylierungs-Status eines Glycoproteins.

**Beispiel 8:**

**Bestimmung der Z von rhu EPO (CHO)**

Die Freisetzung der N-Glycane erfogte aus rhu EPO (CHO) (Boehringer Mannheim) mittels PNGase F in an sich bekannter Weise (Nimtz et al., ibid.).

Die Z wurde analog Beispiel 1 mit Z = 361 bestimmt **(Tabelle 4)**.

Anmerkung:

Von Watson et al. (*Glycobiology* (1994) **4,** 227-237) wurde die Glycosylierung von rhu EPO (CHO) (Amgen) ebenfalls in einer detaillierten analytischen Studie untersucht. Demnach sind die N-Glycane des besagten rhu EPO (CHO) zu über 90% sialyliert, was die im Vergleich zum BHK-EPO aus Beispiel 9 erhöhte Ladungszahl erklärt.

Von Watson et al. (ibid.) wurde der nach PNGase F-Verdau erhaltene N-Glycan-Pool über eine Glycopak™ DEAE-Anionenaustauschersäule der Fa. Waters getrennt. Das in Fig. 1 der Publikation von Watson et al. (S. 228) abgebildete Chromatogram wurde im Rahmen der vorliegenden Erfindung zur Bestimmung der Z nach Gleichung 1 herangezogen. Dabei wurde für das CHO-EPO von Amgen Z = 367 bestimmt, was in sehr guter Übereinstimmung mit der in Beispiel 8 für das CHO-EPO von Boehringer Mannheim bestimmten Ladungszahl von Z = 361 steht.

In der Doktorarbeit von C.H. Hooke (Hokke et al. (1993), in Hokke, C.H. "Structure determination of glycoprotein glycans" (doctoral thesis), pp. 51 - 90) ist die detaillierte Aufklärung der Zuckerstrukturen von rhu EPO (CHO) der Fa. Organon Technika beschrieben. Dabei konnte für dieses EPO gezeigt werden, daß bei 18 - 20 % der N-Glycane ein und bei 3 % der N-Glycane zwei Neuraminsäure-Reste fehlten. Die von Hokke vorgestellten Daten erlaubten im Rahmen der vorliegenden Erfindung die Ermittlung der Z zu Z = 286. Diese Ladungszahl ist gegenüber der Z des CHO-EPO von Amgen (Z = 367) und der Z des CHO-EPO von Boehringer Mannheim (Z = 361), aber auch gegenüber der Z des BHK-EPO von Merckle (Z = 323) deutlich erniedrigt. Aus diesem Grunde darf für das CHO-EPO der Firma Organon Teknika eine deutlich erhöhte Clearance und - damit verbunden - eine deutlich geringere biologische Wirksamkeit angenommen werden.

Anmerkung:

Wie in den vorgegangenen Beispielen und im Eingangstext ausgeführt, kann die Glycosylierung (und somit die Ladungszahl) je nach Gewinnung des entsprechenden Glykoproteins von Charge zu Charge schwanken. Dies wird noch einmal in den Beispielen 9 und 10 belegt.

**Beispiel 9:**

**Z und der Vergleich verschiedener N-Glycan Pools von AGP**

Von Hermentin et al. (*Anal. Biochem.* (1992) **206,** 419-429) wurde ein Vergleich von auf verschiedenem Wege hergestellten N-Glycan Pools von AGP (Charge 281184, Behringwerke AG) vorgenommen und mit einer käuflich erworbenen "N-Glycan Library" von AGP (LB-001, OGS) verglichen. Die jeweils erhaltenen HPAE-PAD-Mapping-Chromatogramme wurden publiziert (Hermentin et al., ibid.). In der genannten Publikation wurde aufgezeigt, daß sich die Mapping Chromatogramme infolge des Verlusts an gebundener N-Acetylneuraminsäure unterscheiden - und dies wurde durch eine Übereinanderlagerung der Mapping-Chromatogramme dokumentiert (Hermentin et al., ibid., Fig. 5).

Im Rahmen der vorliegenden Erfindung wurde für die von Hermentin et al. (ibid., Fig. 5) publizierten Mapping Chromatogramme von AGP retrospektiv die jeweilige Ladungszahl bestimmt (analog Beispiel 1). Dabei ergab sich ein Anstieg der Ladungszahl wie folgt:

| Run a | Z = 248 | (LB-001, OGS; "hydrazinolysis-derived") |
|-------|---------|------------------------------------------|
| Run b | Z = 262 | ("large-scale hydrazinolysis", 50 mg AGP) |
| Run c | Z = 276 | ("large-scale hydrazinolysis", 1000 mg AGP) |
| Run d | Z = 285 | ("automated hydrazinolysis", 2 mg AGP) |
| Run e | Z = 289 | ("PNGase F-derived after previous tryptic AGP digest"). |

Die Z der Läufe belegt mit ihrem jeweiligen Zahlenwert die unterschiedliche Beschaffenheit der Glycan Pools. Sie belegt aber auch den von Hermentin et al. (ibid.) über den Vergleich der Mapping-Chromatogramme erbrachten Befund der Ähnlichkeit der Chromatogramme der Läufe d und e. Dies wiederum belegt die aussagekräftige, hilfreiche und somit vorteilhafte Bedeutung der Ladungszahl Z.

**Beispiel 10:**

**Z und der PNGase F-Verdau von AGP**

Anmerkung:
Von AGP ist bekannt, daß sich seine N-Glycane mit PNGase F ohne vorherigen tryptischen Verdau und/oder den Zusatz spezieller Detergentien nur unvollständig abspalten lassen (Nuck et al. (1990), *Glycoconjugate J.* **7**, 279-286). Im Beispiel 10 wird gezeigt, daß sich die unvollständige Gewinnung des N-Glycan Pools von AGP (bei Verwendung von PNGase F) über die Berechnung der Ladungszahl (analog Beispiel 1) nachweisen läßt:

Hierzu wurden die N-Glycane von AGP (wie in den Beispielen 7 und 8 beschrieben) nach 48-stündiger Inkubation mit PNGase F isoliert. Vom isolierten N-Glycan-Pool wurde die Ladungszahl analog Beispiel 1 zu Z = 248 bestimmt. Dieser Wert liegt deutlich unter dem gemäß Beispiel 9 bestimmten Wert von Z = 289 ("Run e"). Somit läßt sich anhand der Ladungszahlen folgern, daß bei Inkubation von AGP mit PNGase F nach Beispiel 10 (also ohne den in Beispiel 9 verwendeten vorherigen tryptischen AGP-Verdau) offenbar die höher geladenen N-Glycane besonders schwer abgespalten werden.

Dies belegt erneut die aussagekräftige und hilfreiche und somit vorteilhafte Bedeutung der Ladungszahl Z und ihre Bedeutung als diagnostischer Parameter für den Glykosylierungs-Status eines Glykoproteins. Beispielsweise läßt sich durch die Bestimmung der Z von AGP von Einzelspendern eine Aussage über den Grad einer Entzündung machen (De Graf et al., J. Exp. Med. (1993), **177,** 657-666).

**Beispiel 11:**

**Z verschiedener Glycoproteine**

Weil sich Z als ein neuer und sehr hilfreicher und vorteilhafter Meßparameter für die Charakterisierung des Glycosylierungs-Status eines Glycoproteins erweist, sind in **Tabelle 4** die analog Beispiel 1 bestimmten Ladungszahlen verschiedener Glycoproteine beispielhaft aufgeführt. Die Herkunft des jeweiligen Glycoproteins und die Herstellung oder Herkunft des jeweiligen N-Glycan-Pools (Herstellung mittels Hydrazinolyse oder PNGase F oder käuflich erworben bei der Fa. Oxford GlycoSystem (OGS), Abingdon, England) ist ebenfalls aus **Tabelle 4** ersichtlich.

Somit eignet sich die Z in sehr vorteilhafter Weise zur Charakterisierung des Glycosylierungs-Status eines Glycoproteins.

Tabelle 4

| Glycoprotein | Herkunft des Glycoproteins | Herkunft des Glycan Pools | Z |
|---|---|---|---|
| **rhu EPO (CHO)** | Boehringer Mannheim | PNGase F | **361** |
| **rhu EPO (BHK)** | Merckle | PNGase F | **323** |
| **fetales Kälberserum- Fetuin** | Sigma | "large scale"-Hydrazinolyse | **256** |
| **Rinder-Fetuin** | Sigma | GlycoPrep | **290** |
| **Rinder-Pancreas- Ribonuclease B** | | Oxford Glyco Systems (OGS) | **15** |
| **Hühner-Ovomucoid** | | OGS | **15** |
| **Schweine-Thyroglobulin** | | OGS | **82** |
| **humanes alpha-1-saures Glycoprotein** | BW AG (Behringwerke AG) | GlycoPrep | **289** |
| **humanes Serumtransferrin** | | OGS | **207** |
| **humanes Antithrombin III** | BW AG | GlycoPrep | **180** |
| **humanes Fibrinogen** | | OGS | **184** |
| **alpha-1-T-Glycoprotein** | BW AG | GlycoPrep | **187** |
| **alpha-1-Antitrypsin** | BW AG | GlycoPrep | **190** |
| **alpha-1-Antichymotrypsin** | BW AG | GlycoPrep | **236** |
| **β-2-Glycoprotein-I** | BW AG | GlycoPrep | **185** |

Tabelle 4 (fortgesetzt)

| Glycoprotein | Herkunft des Glycoproteins | Herkunft des Glycan Pools | Z |
|---|---|---|---|
| TB6-Glycoprotein | BW AG | GlycoPrep | 208 |
| alpha-1-B-Glycoprotein | BW AG | GlycoPrep | 194 |
| alpha-2-HS-Glycoprotein | BW AG | GlycoPrep | 158 |
| 8S-alpha-3-Glycoprotein | BW AG | GlycoPrep | 145 |
| Haptoglobulin | BW AG | GlycoPrep | 197 |

**Beschreibung der Figuren**

**Figur 1**
Figur 1 zeigt das N-Glycan-Mapping-Profil von rhu IL-4R (Charge E4-930914) nach Trennung mittels HPAE-PAD unter Standardbedingungen nach Hermentin et al., Anal. Biochem. **203** (1992), S. 281-289.
Anmerkung:

S1: Interner Standard 1
S2: Interner Standard 2

Die Glycane befinden sich im Chromatogramm zwischen S1 und S2. Die Peaks vor S1 stammen aus der Hydrazinolyse; die Peaks nach S2 sind unbekannter Natur.
**Figur 2**
Figur 2 zeigt die Fraktionierung von rmur IL-4R (Charge 018PP) mittels Anionenaustausch-Chromatographie an Q-Sepharose FF.
**Figur 3**
Figur 3 zeigt die isoelektrische Focussierung (IEF) der gemäß Figur 2 gewonnenen Q-Sepharose-Fraktionen von rmur IL-4R Charge 018PP.
Die zugehörigen analytischen Daten finden sich in Tabelle 3.
**Figur 4**
Figur 4 a zeigt den Abfall der Z, Figur 4 b den Abfall des NANA-Gehalts von rhu IL-4R im Kulturüberstand bei Lagerung bei Raumtemperatur (RT) + 4 °C, - 20 °C und - 70 °C.

**Patentansprüche**

1. Verfahren zur Charakterisierung der Glycosylierung eines Glycoproteins durch eine hypothetische Ladungszahl (Z), die dadurch erhalten wird, daß man

a) den Glycan-Pool des Glycoproteins isoliert,
b) denselben mittels Ionenaustausch-Chromatographie primär nach Ladung auftrennt,
c) die prozentualen Flächenanteile der ladungsmäßig getrennten Peakgruppen (der Glycane) bestimmt,
d) die prozentualen Flächenanteile der Peakgruppen im neutralen (asialo)-, monosialo- (MS), disialo- (DiS), trisialo- (TriS), tetrasialo-(TetraS) und pentasialo (PentaS)-Bereich mit null (asialo) bzw. 1 (MS) bzw. 2 (DiS) bzw. 3 (TriS) bzw. 4 (TetraS) bzw. 5 (PentaS) multipliziert und
e) über die jeweils erhaltenen Produkte aufsummiert.

2. Verfahren zur *in-vitro* Bestimmung der Bioverfügbarkeit eines Glycoproteins, wobei die für das Glycoprotein mit dem Verfahren gemäß Anspruch 1 erhaltene Ladungszahl in Bezug gesetzt wird zu einem Standardwert, der in einer *in-vivo* Bestimmung erhalten worden ist.

3. Verfahren zur *in-vitro* Bestimmung der Bioverfügbarkeit eines Glycoproteins, wobei die für das Glycoprotein mit dem Verfahren gemäß Anspruch 1) erhaltene Ladungszahl in Bezug gesetzt wird zu einem Standardwert, die rechnerisch erhalten worden ist.

4. Verfahren zur Bestimmung der Chargenkonsistenz eines Glycoproteins, wobei die für das Glycoprotein mit dem Verfahren gemäß Anspruch 1 erhaltene Ladungszahl in Bezug gesetzt wird zu einem Standardwert, der mit dem Verfahren gemäß Anspruch 1 für eine Standardpräparation erhalten worden ist.

5. Verfahren zur Bestimmung der Chargenkonsistenz eines Glycoproteins, wobei die für das Glycoprotein mit dem Verfahren gemäß Anspruch 1 erhaltene Ladungszahl in Bezug gesetzt wird zu einem Standardwert, der rechnerisch erhalten worden ist.

6. Verfahren nach Anspruch 1, **dadurch gekennzeichnet, daß** die Freisetzung des Glycan-Pools aus dem Glycoprotein durch Hydrazinolyse erfolgt.

7. Verfahren nach Anspruch 1, **dadurch gekennzeichnet, daß** die Freisetzung des Glycan-Pools aus dem Glycoprotein auf enzymatischem Wege erfolgt.

8. Verfahren nach Anspruch 1, **dadurch gekennzeichnet, daß** die Ionenaustausch-Chromatographie eine HPAE-PAD (high-pH anion-exchange chromatography with pulsed amperometric detection) ist.

9. Verfahren nach Anspruch 1 zur Charakterisierung des Glycosylierungs-Status eines Glycoproteins.

10. Verfahren nach Anspruch 2 oder 3 zur Überprüfung der Bioverfügbarkeit eines Glycoproteins.

11. Verfahren nach Anspruch 4 oder 5 zur Überprüfung der Chargenkonsistenz eines zelltechnologisch produzierten Glycoproteins.

12. Verwendung eines Verfahrens nach Anspruch 1 als diagnostisches Hilfsmittel bzw. als Diagnostikum

13. Verfahren nach Anspruch 1 zur Überprüfung des Krankheits-Status einer Spezies über deren Glycosylierungs-Status eines für die Erkrankung charakteristischen Glycoproteins.

14. Verfahren nach Anspruch 1 zur Überprüfung des Krankheits-Status eines Patienten über den Glycosylierungs-Status eines für die Erkrankung charakteristischen Glycoproteins.

15. Verfahren nach Anspruch 7, **dadurch gekennzeichnet, daß** die Freisetzung mittels PNGase F erfolgt.

**Claims**

1. A process for characterizing the glycosylation of a glycoprotein by means of a hypothetical charge number (N), which charge number is obtained by

   a) isolating the glycan pool of the glycoprotein,
   b) fractionating the pool, primarily according to charge, by means of ion exchange chromatography,
   c) determining the percentage fractions represented by the areas of the peak groups (the glycans) which have been separated according to charge,
   d) multiplying the percentage fractions represented by the areas of the peak groups in the neutral (asialo), monosialo (MS), disialo (DiS), trisialo (TriS), tetrasialo (TetraS) and pentasialo (PentaS) ranges by zero (asialo), 1 (MS), 2 (DiS), 3 (TriS), 4 (TetraS) and 5 (PentaS), respectively, and
   e) summing the products which are obtained in each case.

2. A process for the *in-vitro* determination of the bioavailability of a glycoprotein, wherein the charge number which has been obtained for the glycoprotein using the process as claimed in claim 1 is related to a standard value which has been obtained in an in-vivo determination.

3. A process for the *in-vitro* determination of the bioavailability of a glycoprotein, wherein the charge number which has been obtained for the glycoprotein using the process as claimed in claim 1 is related to a standard value which has been obtained by calculation.

4. A process for determining the batch consistency of a glycoprotein, wherein the charge number which has been obtained for the glycoprotein using the process as claimed in claim 1 is related to a standard value which has been obtained for a standard preparation using the process as claimed in claim 1.

5. A process for determining the batch consistency of a glycoprotein, wherein the charge number which has been obtained for the glycoprotein using the process as claimed in claim 1 is related to a standard value which has been obtained by calculation.

6. The process as claimed in claim 1, wherein the glycan pool is liberated from the glycoprotein by means of hydrazinolysis.

7. The process as claimed in claim 1, wherein the glycan pool is liberated from the glycoprotein enzymically.

8. The process as claimed in claim 1, wherein the ion exchange chromatography is HPAE-PAD (high-pH anion exchange chromatography with pulsed amperometric detection).

9. The process as claimed in claim 1 for characterizing the glycosylation status of a glycoprotein.

10. The process as claimed in claim 2 or 3 for checking the bioavailability of a glycoprotein.

11. The process as claimed in claim 4 or 5 for checking the batch consistency of a glycoprotein which has been produced by means of cell technology.

12. The use of the process as claimed in claim 1 as a diagnostic aid and/or as a diagnostic agent.

13. The process as claimed in claim 1 for checking the disease status of a species by way of its glycosylation status of a glycoprotein which is characteristic for the disease.

14. The process as claimed in claim 1 for checking the disease status of a patient by way of the glycosylation status of a glycoprotein which is characteristic for the disease.

15. The process as claimed in claim 7, wherein the liberation is effected using PNGase F.

**Revendications**

1. Procédé pour la caractérisation de la glycosylation d'une glycoprotéine au moyen d'un indice de charge hypothétique (Z), qui est obtenu par les opérations consistant à

   a) isoler le groupement glycane de la glycoprotéine,
   b) à effectuer une séparation primaire de celui-ci selon la charge au moyen de la chromatographie par échange d'ions,
   c) à déterminer les proportions de surface en pourcentage des groupes de pics (des glycanes) séparés selon la charge,
   d) à multiplier les proportions de surface en pourcentage des groupes de pics dans le domaine neutre (asialo), monosialo (MS), disialo (DiS), trisialo (TriS), tétrasialo (TétraS) et pentasialo (PentaS) par zéro (asialo) ou 1 (MS) ou 2 (DiS) ou 3 (TriS) ou 4 (Tétras) ou 5 (PentaS) et
   e) à faire la somme sur les produits respectivement obtenus.

2. Procédé pour la détermination *in vitro* de la disponibilité biologique d'une glycoprotéine, dans lequel l'indice de charge obtenu pour la glycoprotéine avec le procédé selon la revendication 1 est mis en rapport avec une valeur de référence, qui a été obtenue dans une détermination *in vivo.*

3. Procédé pour la détermination *in vitro* de la disponibilité biologique d'une glycoprotéine, dans lequel l'indice de charge obtenu pour la glycoprotéine avec le procédé selon la revendication 1 est mis en rapport avec une valeur de référence, qui a été obtenue par le calcul.

4. Procédé pour la détermination de la consistance de la charge d'une glycoprotéine, dans lequel l'indice de charge obtenu pour la glycoprotéine avec le procédé selon la revendication 1 est mis en rapport avec une valeur de référence, qui a été obtenue avec le procédé selon la revendication 1 pour une préparation étalon.

**5.** Procédé pour la détermination de la consistance de la charge d'une glycoprotéine, dans lequel l'indice de charge obtenu pour la glycoprotéine avec le procédé selon la revendication 1 est mis en rapport avec une valeur de référence, qui a été obtenue par le calcul.

**6.** Procédé selon la revendication 1, **caractérisé en ce qu'**on réalise la libération du groupement glycane de la glycoprotéine au moyen d'un hydrazinolyse.

**7.** Procédé selon la revendication 1, **caractérisé en ce qu'**on réalise la séparation du groupement glycane de la glycoprotéine par voie enzymatique.

**8.** Procédé selon la revendication 1, **caractérisé en ce que** la chromatographie par échange d'ions est une HPAE-PAD (high-pH anion-exchange chromatography with pulsed amperometric détection).

**9.** Procédé selon la revendication 1 pour la caractérisation de l'état de glycosylation d'une glycoprotéine.

**10.** Procédé selon la revendication 2 ou 3 pour le contrôle de la biodisponibilité d'une glycoprotéine.

**11.** Procédé selon la revendication 4 ou 5 pour le contrôle de la consistance de charge d'une glycoprotéine produite par technologie cellulaire.

**12.** Utilisation d'un procédé selon la revendication 1 comme adjuvant diagnostique ou comme diagnostic.

**13.** Procédé selon la revendication 1 pour le contrôle de l'état de maladie d'une espèce par l'état de glycosylation d'une glycoprotéine caractéristique de la maladie.

**14.** Procédé selon la revendication 1 pour le contrôle de l'état de maladie d'un patient par l'état de glycosylation d'une glycoprotéine caractéristique de la maladie.

**15.** Procédé selon la revendication 7, **caractérisé en ce que** la libération est réalisée au moyen de la PNGase.

Datei : 094096 K. D 42    Probe :  HY 94138 / 1

FIG.1

3-DiS

2-MS

4-TriS

S1
1-AS/high-Man

5-TetraS

S2

m V

Minuten

EP 0 843 821 B1

rmu IL - 4R

Fraktionierung von rmu IL- 4R durch
Ionenaustauscher - Chromatographie an Q-Sepharese FF

Fraktion Q    1   2   3   4     5

# FIG. 2

# FIG.3

PAGIEF - Muster

018PP

Fraktion Q1

Fraktion Q2

Fraktion Q3

Fraktion Q4

Fraktion Q5

FIG. 4a: Lagerversuch; Ladungszahl Z

-○- RT    -△- +4°C    -◆- 20°C    -▥- -70°C

FIG. 4b: Lagerversuch; NANA-Gehalt (mol/mol)

-○- Rt    -△- +4°C    -◆- 20°C    -▥- -70°C